# EUROPEAN PATENT SPECIFICATION

(11) **EP 3 709 994 B1**
(45) Date of publication and mention of the grant of the patent: **10.09.2025**
(21) Application number: 18815345.6
(22) Date of filing: 13.11.2018
(51) Int. Cl.: A61K 31/375, A61K 31/519, A61K 33/26, A61K 33/34, A23L 33/15, A23L 33/16, A61K 45/06, A61K 9/50, A61P 7/06

(54) **ORAL ADMINISTRATION FORM COMPRISING AN IRON-COPPER MIXTURE AND ITS USE AS A FOOD SUPPLEMENT**
ORALE DARREICHUNGSFORM ENTHALTEND EINE EISEN-KUPFERMISCHUNG UND IHRE VERWENDUNG ALS NAHRUNGSERGÄNZUNGSMITTEL
FORME À ADMINISTRER PAR VOIE ORALE COMPRENANT UN MÉLANGE DE FER-CUIVRE ET SON UTILISATION EN TANT QUE COMPLÉMENT ALIMENTAIRE

(30) Priority: 14.11.2017 IT 201700130043
(43) Date of publication of application: 23.09.2020
(73) Proprietor: Pizeta Pharma S.P.A., 62039 Visso (MC) (IT)
(72) Inventor: CATANESE, Maurizio Danilo, 62039 Visso (MC) (IT); DAL MORO, Giulia, 62039 Visso (MC) (IT)
(74) Representative: Villa, Livia
(86) International application number: PCT/IB2018/058919
(87) International publication number: WO 2019/097400

(56) References cited:
- CN-A- 101 912 049
- CN-B- 103 271 940
- GB-A- 2 212 396
- US-A1- 2009 035 385
- US-B1- 6 488 956

## Description

### FIELD OF THE INVENTION

The present invention relates to an oral administration form comprising an association of Fe(II) and Cu(II). The oral administration form finds advantageous application as a food supplement for the supplementation of iron and copper, without side effects for the stomach and intestine.

### BACKGROUND ART

Iron-deficiency anaemia or sideropenia is a condition in which the body's total iron level is reduced considerably, thus compromising the iron supply to all tissues, but in particular to erythropoiesis.

Iron-deficiency anaemia is the most common form of anaemia worldwide. The causes of iron-deficiency are well known. Some are physiological, such as the increased need for iron in childhood, during body growth, in the third trimester of pregnancy, or during breastfeeding. Others include insufficient dietary intake, reduced intestinal absorption, or chronic blood loss. Blood loss may occur for different reasons: excessive menstrual loss is to be considered in women of childbearing age, metrorrhagia, and repeated pregnancies not sufficiently offset by martial therapy. In both sexes, but especially among the elderly, hidden blood loss via the gastrointestinal tract is also frequent as a result of benign diseases, iatrogenic complications, or neoplasia. Repeated blood donations may lead to iron deficiency. Reduced absorption may result from atrophic gastritis, chronic infection with *Helicobacter pylori,* celiac disease, often in atypical form, and may onset following duodenum bypass surgery or surgery resulting in the reduction of gastric acidity.

Iron deficiency is treated with iron administered orally, typically in doses corresponding to 100-200 mg of elemental iron. The most bioavailable form of iron is represented by ferrous salts, in particular, ferrous sulphate.

However, some patients are unable to tolerate oral treatment because of the side effects thereof, especially in the intestine (nausea, vomiting, constipation), or they have reduced compliance and must therefore be treated with parenteral iron. Chronic atrophic gastritis, which sometimes leads to *H. pylori* infection, also causes reduced absorption and said type of gastritis is often not reversible after eradication of the infection.

Recent studies suggest that poor tolerance of iron administered orally may be induced by unabsorbed iron, which could damage the intestinal mucosa due to the oxidising properties thereof. Furthermore, some studies suggest that excess iron in the intestine is harmful to the local flora, determining a change in the microbiota and the replacement of saprophytic species with pathogenic species.

There are also patients with poor absorption attributable to intestinal causes or treatment with pump inhibitors, which can be refractory to oral treatment.

One metal that is directly involved in the absorption and transport of iron is copper. Copper deficiency alters the role of other cellular constituents involved in antioxidant activities, such as iron, selenium, and glutathione, and consequently plays an important role in diseases in which oxidative stress is high and where there is reduced absorption of iron and impaired thyroid functioning.

GB2212396 discloses a dietary calcium and iron supplement for oral ingestion comprising a nutritionally effective amount of a composition comprising iron, a delayed release coating surrounding said iron composition which delays release of said iron from said coating, and a nutritionally effective amount of a substantially immediate release composition comprising calcium. After the supplement reaches the stomach, the calcium is rapidly available for entry into the small intestine while the release of the iron in the small intestine is delayed for at least one hour after the entry of the calcium into the small intestine. As a result, calcium and iron can be simultaneously delivered without adverse mineral interactions.

US2009/035385A1 discloses nutritional or dietary supplement compositions with improved iron solubility that promote dietary iron absorption through the administration of iron with one or more organic acids, as iron absorption promoters, such as succinic acid, malic acid and tartaric acid. Other suitable iron absorption promoters include for example but are not limited to ascorbic acid, salts of ascorbic acid, derivatives of ascorbic acid, compounds having Vitamin C activity, carbohydrates such as but not limited to mannitol, sorbitol, xylose, inositol, fructose, sucrose, lactose, and glucose, calcium, copper, sodium molybdate, amino acids and combinations thereof.

The object of the present invention is therefore to provide a product which effectively treats iron deficiency, while also overcoming the drawbacks of oral iron administration.

### SUMMARY OF THE INVENTION

Said object has been achieved by an oral administration form comprising Fe(II) and Cu(II), as stated in Claim 1.

In another aspect, the present invention relates to the use of said oral administration form for the treatment of iron deficiency.

In another aspect, the present invention relates to a unit dose of said oral administration form comprising Fe(II) and Cu(II).

In another aspect, the present invention relates to a food supplement comprising said oral administration form.

As will be clear by the following detailed description and working Examples provided for illustrative and non-limiting purposes, the oral administration form of the invention has surprisingly proved to be highly effective for supplementing iron in its most bioavailable form, namely Fe(II), while also offering optimal tolerability by the body, even over long periods of administration.

### BRIEF DESCRIPTION OF THE FIGURES

The characteristics and advantages of the present invention will become apparent from the following detailed description, working Examples provided for illustrative and non-limiting purposes and figures annexed hereto, wherein:
- Figure 1 shows the Fe content of formulations in the respective supernatant portions and pellets, according to Example 7,
- Figure 2 shows the concentration of Fe present in the apical (luminal) compartment at the beginning and at the end of the absorption step (3 hours, *p<0.05), according to Example 7, and
- Figure 3 shows the levels of ferritin present in the cells constituting the intestinal epithelium model following exposure to the three formulations in Example 7 (*p<0.05).

### DETAILED DESCRIPTION OF THE INVENTION

Therefore, the invention relates to an oral administration form comprising: a) an active core comprising a solid association of at least one pharmaceutically acceptable Fe(II) salt and at least one pharmaceutically acceptable Cu(II) salt and (b) an active core-coating layer, said coating layer comprising at least one enteric polymer and at least one hydrophilic polymer, as stated in Claim 1.

Divalent iron is the most bioavailable form because said form is absorbed by enterocytes. However, divalent iron irritates both the gastric mucosa and the intestinal mucosa. This irritation also has a negative effect on the absorption of said iron, to such an extent that long-term treatment is rendered ineffective.

It has unexpectedly been found that, by formulating the Fe(II) according to the present invention, it is possible to benefit from all the advantages of Fe(II) supplementation, but without experiencing the side effects stated above. Indeed, the coating layer effectively masks the typical taste of iron, offers long-term stability and, above all, prevents contact between the iron and the gastric walls, since it releases the iron directly within the enterocytes during fasting and onto the ileum in the small intestine.

At the same time, the possibility of using Fe(II) also overcomes the disadvantages associated with the use, as an alternative, of Fe(III), wherein said disadvantages usually manifest through an accumulation of iron in the liver. Indeed, the layer coating the oral administration form the invention also protects the Fe(II) present therein, advantageously preventing its oxidation to Fe(III).

Furthermore, the oral administration form is particularly stable as said form may be kept at room temperature for at least 24 months.

With the term "solid association", it is understood that said at least one pharmaceutically acceptable Fe(II) salt and at least one pharmaceutically acceptable Cu(II) salt are mixed together to form a solid mixture or are present separately in the active core, both in the solid state. In fact, as explained in more detail when describing the preparation process, said salts may be pre-mixed together or added separately.

For the purposes of the present invention, suitable pharmaceutically acceptable salts of Fe(II) include both inorganic salts and complex compounds of Fe(II), as well as mixtures thereof.

Preferably, said at least one pharmaceutically acceptable Fe(II) salt is ferrous sulphate, ferrous fumarate, ferrous gluconate, ferrous succinate, ferrous glutamate, ferrous lactate, ferrous citrate, ferrous tartrate, ferrous pyrophosphate, ferrous carbonate, ferrous chloride, ferrous iodide, ferrous bisglycinate, ferrous saccharate, ferrous phosphate, ferrous formate, ferrous acetate, ferrous malate, ammonium ferrous sulphate, ferrous hydroxide, ferrous nitrate, ferrous oxalate, or a mixture thereof.

More preferably, said at least one pharmaceutically acceptable Fe(II) salt is ferrous fumarate, ferrous gluconate, ferrous succinate, ferrous lactate, ferrous citrate, ferrous tartrate, ferrous bisglycinate, ferrous oxalate, or a mixture thereof.

In particularly preferred embodiments, said at least one pharmaceutically acceptable Fe(II) salt is ferrous fumarate.

In the oral administration form of the invention, said at least one pharmaceutically acceptable Fe(II) salt is in the form of micronised particles having a particle size distribution D₉₀ of 1-50 µm. For the purposes of the present invention, this parameter is measured through particle size analysis by sieving with suitable sieves arranged in series, each one of which retains the solid moiety whose granules are larger in size than the sieve holes. Preferably, said at least one pharmaceutically acceptable Fe(II) salt is in the form of micronised particles having a particle size distribution D₉₀ of 2-20 µm, more preferably 3-7 µm, and even more preferably of 5 µm.

For the purposes of the present invention, suitable pharmaceutically acceptable salts of Cu(II) include both inorganic salts and complex compounds of Cu(II), as well as mixtures thereof.

Preferably, said at least one pharmaceutically acceptable salt of Cu(II) is copper gluconate, copper citrate, copper lauroyl sarcosinate, copper formate, copper acetate, copper propionate, copper butyrate, copper lactate, copper oxalate, copper phytate, copper tartrate, copper malate, copper succinate, copper malonate, copper maleate, copper benzoate, copper salicylate, copper aspartate, copper glutamate, copper fumarate, copper glycerophosphate, chlorophyllin sodium copper, copper chloride, copper fluoride, copper sulphate, copper fluorosilicate, copper nitrate, copper fluoroborate, copper iodate, or a mixture thereof.

More preferably, said at least one pharmaceutically acceptable Cu(II) salt is copper gluconate, copper citrate, copper succinate, copper salicylate, copper aspartate, or a mixture thereof.

In particularly preferred embodiments, said at least one pharmaceutically acceptable Cu(II) salt is copper gluconate.

Preferably, in the oral administration form the invention, said at least one pharmaceutically acceptable Fe(II) salt is present in a greater amount than said at least one pharmaceutically acceptable Cu(II) salt.

In preferred embodiments of the invention, said at least one pharmaceutically acceptable Fe(II) salt and said at least one pharmaceutically acceptable Cu(II) salt are in a weight ratio of 200: 1 to 2:1. Preferably, said at least one pharmaceutically acceptable Fe(II) salt and said at least one pharmaceutically acceptable Cu(II) salt are in a weight ratio of 100: 1 to 10:1, more preferably 50:1 to 20:1.

In some embodiments, the oral administration form the invention comprises up to 10wt% of said at least one pharmaceutically acceptable Fe(II) salt, preferably up to 5wt%.

For the purposes of the present invention, unless otherwise specified, "wt%" means % of weight, based on the weight of the oral administration form of the invention.

In other embodiments, the oral administration form of the invention comprises up to 1wt% of said at least one pharmaceutically acceptable Cu(II) salt, preferably up to 0.1wt%.

The oral administration form of the invention can further comprise also pharmacologically acceptable excipients. The term "excipient" means a compound or a mixture thereof suitable for use in a formulation for the treatment of iron deficiency or conditions associated thereto. For example, an excipient for use in a pharmaceutical formulation should not generally cause an adverse response in a patient, nor should it significantly inhibit the efficacy of said formulation. Suitable excipients are diluents, disaggregating agents, glidants, binders, lubricants, stabilisers, adsorbents, release retardants, flavourings, sweeteners, anti-caking agents, and preservatives. In particular, they are used for the purposes of the present invention, natural starch, partially hydrolysed starch, lactose, glucose, sucralose, fructose, sucrose, mannitol, sorbitol, cellulose and derivatives thereof, microcrystalline cellulose and derivatives thereof, calcium phosphate, calcium carbonate, calcium sulphate, gelatin, tragacanth gum, gum arabic, polyethylene glycol, alginates, talc, silica, colloidal silica, precipitated silica, magnesium silicates, aluminium silicates, sodium lauryl sulphate, magnesium lauryl sulphate, methacrylate copolymers, potassium citrate tribasic monohydrate, polysorbate 20, polysorbate 40, polysorbate 60, polysorbate 80, esters of fatty acids, monoglycerides of fatty acids, diglycerides of fatty acids, and mixtures thereof.

Said at least one enteric polymer is a polymer capable of passing through the gastric tract, without, therefore, experiencing degradation in the presence of a strongly acidic pH), and then disintegrating once it has reached the intestinal tract with a basic pH, thus releasing the active ingredients contained therein.

The enteric polymers of the present invention are cellulose acetate phthalate, cellulose acetate succinate, hydroxypropyl methylcellulose phthalate, hydroxypropyl methylcellulose acetate succinate, polyvinyl acetate phthalate, hydroxyethyl cellulose phthalate, cellulose acetate tetrahydro phthalate, copolymers of methacrylate-methacrylic acid, copolymers of methyl methacrylate- methacrylic acid, sodium alginate, cellulose acetate trimellitate, shellac, or a mixture thereof.

Preferably, said at least one enteric polymer is shellac.

Said at least one hydrophilic polymer is a polymer capable of delaying the release of the active ingredients.

The hydrophilic polymers of the present invention are hydroxypropyl methylcellulose, hydroxypropyl cellulose, hydroxyethyl cellulose, xanthan gum, cross-linked carboxy methyl cellulose and the salts thereof, polyvinyl alcohol, polyvinylpyrrolidone, or a mixture thereof.

Preferably, said at least one hydrophilic polymer is hydroxypropyl cellulose, hydroxypropyl methylcellulose, polyvinylpyrrolidone, or a mixture thereof.

In preferred embodiments, said oral administration form furthermore comprises an inert agent selected from talc, corn starch, lactose, microcrystalline cellulose, polyethylene glycol, and mixtures thereof. Preferably, said oral administration form furthermore comprises talc and corn starch.

Preferably, said oral administration form is a capsule, tablet, mini-tablet, micro-tablet, granules, microgranules, pellet, multi-particulate, or micronised particulate.

Preferably, said oral administration form is orodispersible.

More preferably, said oral administration form is microgranules.

Preferably, said microgranules have a D₅₀ median particle-size distribution of 200-800 µm. For the purposes of the present invention, this parameter is measured through particle size analysis by sieving with suitable sieves arranged in series, each one of which retains the solid moiety whose granules are larger in size than the sieve holes.

In preferred embodiments, said microgranules have a D₅₀ median particle-size distribution of 250-600 µm.

The thickness of the coating layer is preferably 100-300 µm, more preferably 100-200 µm, and even more preferably of 100-500 µm.

The weight ratio of the active core to the coating layer is preferably of 6:1 to 1:1, more preferably 3:1 to 1:1, and even more preferably 2:1 to 1:1.

The oral administration form of the invention further comprises at least one vitamin being at least vitamin C, since the latter facilitates the body's absorption of iron. Suitable other vitamins are vitamin A, vitamin B1, vitamin B2, vitamin B6, vitamin B3, vitamin B9, vitamin B12, vitamin D, vitamin E, and vitamin K.

Preferably, the oral administration form of the invention comprises up to 15wt% of vitamin C, and more preferably up to 10wt%.

In some embodiments, the oral administration form of the invention further comprises folic acid, lipoic acid, lactoferrin or a mixture thereof.

In other embodiments, the oral administration form of the invention further comprises folic acid.

Preferably, the oral administration form of the invention comprises up to 0.2wt% of folic acid, and more preferably up to 0.1wt%.

In other embodiments, the oral administration form of the invention further comprises lipoic acid.

Preferably, the oral administration form of the invention comprises up to 15wt% of lipoic acid, and more preferably up to 10wt%.

In other embodiments, the oral administration form of the invention further comprises lactoferrin.

Preferably, the oral administration form of the invention comprises up to 15wt% of folic acid, and more preferably up to 10wt%.

In preferred embodiments, in the oral administration form of the invention, the active core a) comprises an internal core comprising at least one vitamin, coated with a first layer of at least one hydrophilic polymer and a second layer of a solid mixture of at least one pharmaceutically acceptable Fe(II) salt and at least one pharmaceutically acceptable Cu(II) salt. Preferably, the active core a) comprises an internal core comprising at least one vitamin, coated with a first layer of at least one hydrophilic polymer and an inert agent and a second layer of a solid mixture of at least one pharmaceutically acceptable Cu(II) salt and at least one pharmaceutically acceptable Fe(II) salt and at least one enteric polymer; and the coating layer b) comprises at least one enteric polymer, at least one hydrophilic polymer and at least one inert agent.

In other embodiments, in the oral administration form the invention, the active core a) comprises an internal core comprising at least one vitamin, coated with a first layer of at least one hydrophilic polymer, a second layer of at least one pharmaceutically acceptable Cu(II) salt, and a third layer of at least one pharmaceutically acceptable Fe(II) salt. Preferably, the active core a) comprises an internal core comprising at least one vitamin, coated with a first layer of at least one hydrophilic polymer and an inert agent, a second layer of at least one pharmaceutically acceptable Cu(II) salt, and a third layer of at least one pharmaceutically acceptable Fe(II) salt and at least one enteric polymer; and the coating layer b) comprises at least one enteric polymer, at least one hydrophilic polymer and at least one inert agent.

In further preferred embodiments, in the oral administration form the invention, the active core a) comprises an internal core comprising at least one vitamin, coated with a first layer comprising at least one hydrophilic polymer and at least one pharmaceutically acceptable Fe(II) salt, and a second layer comprising at least one hydrophilic polymer and at least one pharmaceutically acceptable Cu(II) salt. Preferably, the active core a) comprises an internal core comprising at least one vitamin, coated with a first layer comprising at least one hydrophilic polymer and at least one pharmaceutically acceptable Fe(II) salt, a second layer of an inert agent, and a third layer comprising at least one hydrophilic polymer and at least one pharmaceutically acceptable Cu(II) salt.

In other preferred embodiments, in the oral administration form the invention, the active core a) comprises an internal core comprising at least one vitamin, coated with a first layer comprising at least one hydrophilic polymer and at least one pharmaceutically acceptable Cu(II) salt, and a second layer comprising at least one hydrophilic polymer and at least one pharmaceutically acceptable Fe(II) salt. Preferably, the active core a) comprises an internal core comprising at least one vitamin, coated with a first layer comprising at least one hydrophilic polymer and at least one pharmaceutically acceptable Cu(II) salt, a second layer of an inert agent, and a third layer comprising at least one hydrophilic polymer and at least one pharmaceutically acceptable Fe(II) salt.

Preferably, said embodiments are in the form of microgranules.

It should be understood that the preferred aspects specified above for the individual components should likewise be considered as preferred also for these preferred embodiments.

In other embodiments, the oral administration form the invention does not comprise an internal core of inert agents.

In some embodiments, the oral administration form of the invention consists essentially of a) an active core comprising a solid association of at least one pharmaceutically acceptable Fe(II) salt and at least one pharmaceutically acceptable Cu(II) salt and optionally at least one vitamin, and (b) an active core- coating layer, said coating layer comprising at least one enteric polymer and at least one hydrophilic polymer. The expression "consists essentially of" means that said at least one pharmaceutically acceptable Fe(II) salt, said at least one pharmaceutically acceptable Cu(II) salt, and said optional at least one vitamin, are the only components present in the oral administration form of the invention which are active in the treatment of iron deficiency, the others being formulating components or pharmaceutically acceptable excipients which do not interfere with the action of Fe(II) and Cu(II).

In further embodiments, the oral administration form of the invention consists of a) an active core comprising a solid association of at least one pharmaceutically acceptable Fe(II) salt and at least one pharmaceutically acceptable Cu(II) salt and optionally at least one vitamin, and (b) an active core-coating layer, said coating layer comprising at least one enteric polymer and at least one hydrophilic polymer.

It should also be understood that all aspects identified as favourable and advantageous for the oral administration form of the invention should be deemed to be similarly preferable and advantageous also for these embodiments.

In preferred embodiments, the oral administration form of the invention is in the form of a unit dose.

Preferably, said unit dose comprises up to 200 mg of at least one pharmaceutically acceptable Fe(II) salt, more preferably 10-150 mg.

In particularly preferred embodiments, said unit dose comprises 50-100 mg of at least one pharmaceutically acceptable Fe(II) salt.

Preferably, said unit dose comprises up to 10 mg of at least one pharmaceutically acceptable CU(II) salt, more preferably 0.1-5 mg.

In particularly preferred embodiments, said unit dose comprises 0.5-2 mg of at least one pharmaceutically acceptable Cu(II) salt.

Preferably, said unit dose further comprises up to 300 mg of at least one vitamin, more preferably 50-200 mg.

Preferably, said unit dose further comprises 10-1500 µg of folic acid, more preferably 50-1000 µg.

In preferred embodiments, said unit dose comprises up to 200 mg of at least one pharmaceutically acceptable Fe(II) salt, up to 10 mg of at least one pharmaceutically acceptable Cu(II) salt, up to 300 mg of at least one vitamin, and optionally 10-1500 µg of folic acid.

In particularly preferred embodiments, said unit dose comprises up to 200 mg of Fe(II) fumarate, up to 10 mg of CU(II) gluconate, up to 300 mg of vitamin C, and 10-1500 µg of folic acid.

In particularly preferred embodiments, in said unit dose, the active core a) comprises an internal core comprising at least one vitamin, coated with a first layer of at least one hydrophilic polymer and a second layer of a solid association of at least one pharmaceutically acceptable Fe(II) salt and at least one pharmaceutically acceptable Cu(II) salt. Preferably, said unit dose embodiments are in the form of microgranules. Particularly preferred are the unit dose embodiments in the form of microgranules, wherein a) the active core comprises an internal core comprising vitamin C, coated with a first layer of at least one hydrophilic polymer and a second layer of a solid association of Fe(II) fumarate and Cu(II) gluconate, and b) an active core-coating layer having a thickness of 100-300 µm, said coating layer comprising shellac and ethylcellulose, hydroxypropyl cellulose, hydroxypropyl methylcellulose, polyvinylpyrrolidone, or a mixture thereof.

It should be understood that the preferred aspects specified for the individual components should likewise be considered as preferred in the unit dose embodiments stated above. The oral administration form of the present invention, as well as the unit dose thereof, may be prepared by methods known in the art.

In preferred embodiments, the oral administration form of the invention is obtained by a preparation process comprising the following steps:
i) providing a core comprising at least one vitamin,
ii) coating said core with a first layer of at least one hydrophilic polymer, optionally comprising at least one inert agent,
iii) coating the coated core from step ii) with a second layer of at least one pharmaceutically acceptable Fe(II) salt and at least one pharmaceutically acceptable Cu(II) salt, thus obtaining an active core, and
iv) coating the active core thus obtained with a coating layer comprising at least one enteric polymer and at least one hydrophilic polymer, optionally comprising at least one inert agent, thereby obtaining the oral administration form of the invention.

Preferably, said oral administration forms are microgranules, more preferably orodispersible microgranules.

Preferably, in step ii), said at least one hydrophilic polymer is dissolved in a hydroalcoholic solution before being applied to the core from step i).

Preferably, in step iii), said solid association is preliminarily dissolved in a suitable solvent and subsequently sprayed onto the core coated in step ii). In preferred embodiments, said at least one pharmaceutically acceptable Cu(II) salt applied prior to said at least one pharmaceutically acceptable Fe(II) salt. Preferably, said suitable solvent is an alcoholic solution of at least one enteric polymer.

Preferred solvents are ethanol and water-ethanol solutions.

In another aspect, the present invention relates to the oral administration form described above for use in the treatment of iron deficiency. As mentioned, the oral administration form of the invention has, surprisingly, allowed to benefit from all the advantages of Fe(II) supplementation, but without experiencing the side effects stated above. Indeed, the coating layer effectively masks the typical taste of iron, is extremely stable over time, and above all prevents contact between iron and the gastric walls, since it releases the iron directly within the enterocytes.

Given the advantages found, in addition to the high tolerability by the body, the oral administration form of the invention is particularly recommended for iron supplementation in children, pregnant women, and the elderly.

In another aspect, the present invention relates to a food supplement comprising the oral administration form described above.

More preferably, the food supplement of the invention is administered orally.

In preferred embodiments, the food supplement of the invention is administered in doses of 1-5 g per day, said dose comprising up to 200 mg of at least one pharmaceutically acceptable Fe(II) salt.

The food supplement of the invention finds advantageous use in iron supplementation, because:
- Fe(II) contributes to normal cognitive function, to normal energy metabolism, to the normal formation of red blood cells and haemoglobin, to the normal transportation of oxygen within the body, to the normal functioning of the immune system, to reducing tiredness and fatigue and to normal cognitive development in children, while intervening in the cell division process;
- Cu(II) contributes to the maintenance of normal connective tissues, to normal energy metabolism, the normal functioning of the nervous system, normal pigmentation of hair, the normal iron transport in the body, normal pigmentation of skin, the normal functioning of the immune system, and the protection of cells from oxidative stress;
- vitamin C, when present, increases absorption of iron, contributes to the normal formation of collagen for the normal function of blood vessels, to normal collagen formation for normal bone function, to normal collagen formation for normal cartilage function, to normal collagen formation for normal gingival function, normal collagen formation for normal skin function, normal training collagen for normal tooth function, to normal energy metabolism, to normal function of the nervous system, to normal psychological function, to normal function of the immune system, to the protection of cells from oxidative stress, the reduction of tiredness and fatigue, and to the regeneration of the reduced form of vitamin E.

It should be understood that all the possible combinations of the preferred aspects of the oral administration form, the unit doses, the preparation process, and the uses of said food supplement, the preparation process and the use of said oral administration form are disclosed herein and therefore are also preferred.

It should also be understood that all aspects identified as preferred and advantageous for the oral administration form and the components thereof, should be deemed to be similarly preferable and advantageous also for the uses of said food supplement, and the preparation processes and uses of said oral administration form.

Below are working examples of the present invention provided for illustrative purposes.

### EXAMPLES

### Example 1.

A 1.5 g oral administration form was prepared comprising iron fumarate (wherein 30 mg Fe(II)), copper gluconate (wherein 1 mg Cu(II)), 80 mg vitamin C, talc, fructose, flavouring agent, sucralose, silicon dioxide, shellac, hydroxypropyl cellulose, and hydroxypropyl methylcellulose.

A vitamin C core was prepared, which was subsequently coated with a first layer of hydroxypropyl methylcellulose and talc, starting from a water-ethanol solution.

A second layer was applied to the core thus coated by spraying copper gluconate in ethanol and shellac.

A third layer was then applied by spraying iron fumarate in ethanol and shellac.

In this manner, the active core was obtained.

Finally, said active core was coated with a coating layer comprising shellac, hydroxypropyl cellulose, and talc, thus obtaining the oral administration form of the invention.

### Example 2.

The oral administration form in Example 1 was subjected to an *in vitro* test called *"Dissolution tests for solid oral forms*" according to the European Pharmacopoeia, to assess the release of Fe(II):

| Analysis | Result |
|---|---|
| In HCl 0.1M solution: | |
| • after 0.5 h | 31.9% |
| • after 1 h | 54.9% |
| • after 2 h | 58.6% |
| In a pH 6.8 solution: | |
| • after 3 h | 53.4% |
| In a pH 8 solution: | |
| • after 2 h | > 82% |

### Example 3.

A 1.5 g oral administration form was prepared comprising iron fumarate (wherein 30 mg Fe(II)), copper gluconate (wherein 1 mg Cu(II)), 80 mg vitamin C, 400 µg folic acid, talc, fructose, flavouring agent, sucralose, silicon dioxide, shellac, hydroxypropyl cellulose, and hydroxypropyl methylcellulose.

A vitamin C core was prepared, which was subsequently coated with a first layer of hydroxypropyl methylcellulose and talc, starting from a water-ethanol solution.

A second layer was applied to the core thus coated by spraying copper gluconate, iron fumarate, and folic acid in ethanol and shellac.

In this manner, the active core was obtained.

Finally, said active core was coated with a coating layer comprising shellac, hydroxypropyl cellulose, and talc, thus obtaining the oral administration form of the invention.

### Example 4 (not according to the claimed invention).

A 1.5 g oral administration form was prepared comprising iron fumarate (wherein 40 mg Fe(II)), copper gluconate (wherein 0.1 mg Cu(II)), 20 mg vitamin C, fructose, flavouring agent, sucralose and silicon dioxide.

The layer of coating comprised ethyl cellulose and cellulose acetate succinate, thus obtaining the oral administration form of the invention, according to the procedure in Example 1.

### Example 5.

A 1.5 g oral administration form was prepared comprising iron oxalate (wherein 35 mg Fe(II)), copper aspartate (wherein 2 mg Cu(II)), 100 mg vitamin C, corn starch, fructose, flavouring, sucralose, silicon dioxide , shellac, hydroxymethyl cellulose and hydroxypropyl methylcellulose.

A vitamin C core was prepared, which was subsequently coated with a first layer of hydroxymethyl cellulose and corn starch, starting from a water-ethanol solution.

A second layer was applied to the core thus coated by spraying copper gluconate and iron fumarate in ethanol, with hydroxymethyl cellulose and hydroxypropyl methylcellulose. In this manner, the active core was obtained.

Finally, said active core was coated with a coating layer comprising shellac, hydroxypropyl cellulose, and talc, thus obtaining the oral administration form of the invention.

### Example 6.

A 1.5 g oral administration form was prepared comprising iron lactate (wherein 35 mg Fe(II)), copper succinate (wherein 2 mg Cu(II)), 100 mg vitamin C, fructose, flavouring agent, sucralose, and silicon dioxide.

The layer of coating comprised shellac and hydroxypropyl methylcellulose, thus obtaining the oral administration form of the invention, according to the procedure in Example 3.

### Example 7.

A 1.5 g oral administration form was prepared comprising iron fumarate (wherein 30 mg Fe(II)), copper gluconate (wherein 1 mg Cu(II)), 80 mg vitamin C, talc, shellac, hydroxypropyl cellulose, and hydroxypropyl methylcellulose.

A vitamin C core was prepared, which was subsequently coated with a first layer of hydroxypropyl methylcellulose, iron fumarate, and talc, starting from a water-ethanol solution.

A second layer was applied to the core thus coated by spraying copper gluconate in ethanol and shellac.

In this manner, the active core was obtained.

Finally, said active core was coated with a coating layer comprising shellac, hydroxypropyl cellulose, and talc, thus obtaining the oral administration form of the invention.

### Example 8.

### Comparative assessment of intestinal iron absorption

A study was carried out in order to assess intestinal and intracellular absorption of the iron released by oral administration forms of the present invention, comparing said absorption with that of comparative formulations.

To this end, the following formulations were provided:
1) granular administration form of Example 7 of the invention (shortly: Invention Granule),
2) granular administration form of Example 7 but without copper gluconate (shortly: Control Granule),
3) solid mixture of the ingredients listed in Example 7 (shortly: Mixture).

In order to assess Fe release, formulations 1-3 were treated as follows: equal amounts of the different formulations were resuspended in hydrochloric acid (HCl) 0.1 M (pH 1.2) and left under stirring for 2 hours at 37 °C; next, the different suspensions were diluted in culture medium devoid of FBS (Fetal Bovine Serum) and, after neutralising the pH thereof (final pH: 6.8), were left under stirring at 37 °C for a further 3 hours.

### In vitro model of intestinal epithelium

Absorption of Fe was determined by using an *in vitro* model of human intestinal epithelium based on human Caco-2 intestinal adenocarcinoma cells, organised as a functional monolayer on Transwell^{®} inserts. Briefly, Transwell^{®} inserts featured two compartments, apical (or luminal) and basolateral (or serosal), separated by a microporous membrane. Caco-2 monolayers cultivated on microporous membranes were made up of polarised cells with the typical morpho-functional features of enterocytes, such as the presence of microvilli, occluding junctions (tight junctions) and glycoprotein-p (P-gp).

### Assessment of intestinal absorption of Fe

The bioaccessible fractions of formulations 1-3 were added to the apical compartment of the *in vitro* intestinal epithelium, while the FBS-free cellular medium was added to the basolateral compartment. After 3 hours of incubation, the apical fractions and the Caco-2 cell monolayers were recovered and the respective Fe content determined by ICP-MS analysis.

### Determination of intracellular ferritin

At the end of the 3 hours of exposure, the Caco-2 cell monolayers were subjected to repeated washings in order to remove the unabsorbed Fe, and left to incubate for a further 21 hours in cellular medium (1% FBS) [Scheers NM, et al., "Proposing a Caco-2/HepG2 cell model for in vitro ironabsorption studies", J NutrBiochem., 2014], in order to allow expression of ferritin, a protein involved in the storage of Fe. Once incubation has ended, the Caco-2 cells were resuspended by trypsin-EDTA treatment, pelleted and sonicated in an appropriate buffer. The ferritin content of the various cellular lysates was determined by ELISA (Enzyme-LinkedImmunoSorbentAssay), using a commercial kit.

### RESULTS

### Release of Fe from the three formulations - "bio-accessible fractions"

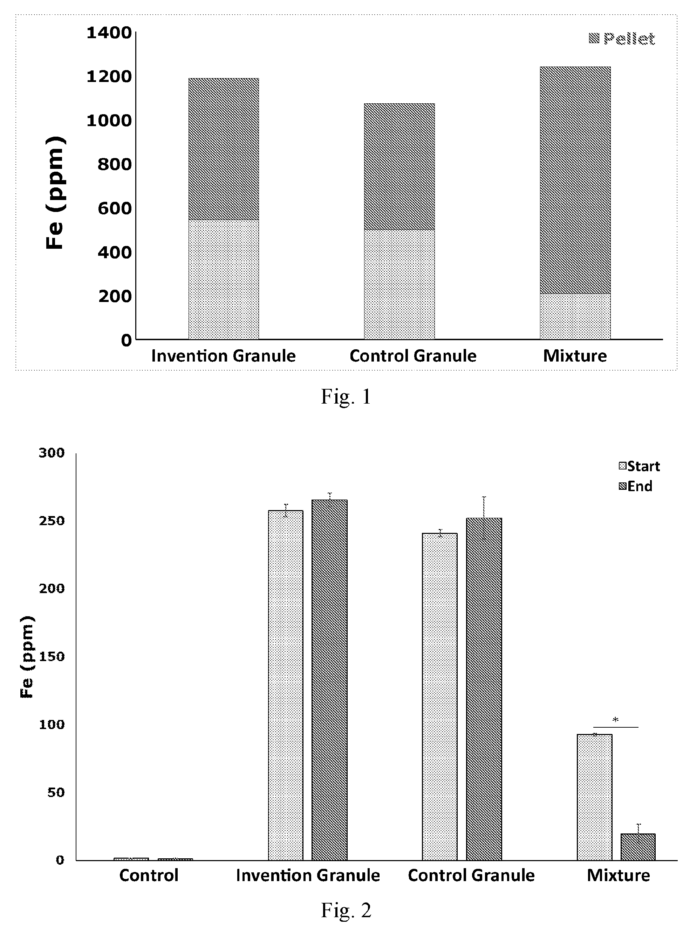

At the end of the treatment step disclosed above (incubation of the three formulations in HCl and subsequent pH neutralisation), the total Fe concentration was similar among Formulations 1-3 and compatible with the expected formulation (approx. 1200 ppm). As illustrated in Figure 1, most of the Fe present in the Mixture formulation is located in the pellets (1030.6 ppm corresponding to 83.1% of total Fe), indicating a poor propensity of the mixture to release Fe in a bio-accessible form (only 16.9% of total Fe was present in the supernatant).

The Control Granule formulation has a concentration of Fe in the supernatant of 498.8 ppm, indicating that this form of administration promotes the release of Fe.

Surprisingly, the Invention Granule formulation further increased the concentration of Fe in the supernatant, since a value of 544.1 ppm was observed, i.e. an increase of approximately 10% compared to the formulation in granules without Cu.

### Intestinal absorption of Fe

In order to assess Fe absorption, intestinal epithelia were exposed for 3 hours to the bio-accessible fractions of Formulations 1-3. At the end of the exposure period, Fe levels were monitored in the apical (lumen) and intracellular fractions.

As can be seen from Figure 2, the bio-accessible fraction of Fe derived from the Mixture showed a significant drop in apical (luminal) Fe during period of exposure, mainly due to precipitation phenomena.

Differently, the bio-accessible fractions of granule formulations are stable over time, even if the Invention Granule fraction resulted to be more stable.

Intracellular absorption of Fe released from the three formulations was assessed by determining the quantity of micronutrient and ferritin in the enterocytes. More specifically, ferritin was selected as an indicator of intracellular iron content as involved in the Fe storage process and its expression depends on intracellular Fe levels.

The intracellular Fe concentration of the Invention Granule and Control Granule formulations was approximately 10 times greater than in the untreated control. The amount of Fe measured in the intestinal epithelium, however, was not representative of the actual intracellular content of the micronutrient, due to the precipitation phenomenon described above, which leads to an aspecific, hard-to-remove Fe deposit.

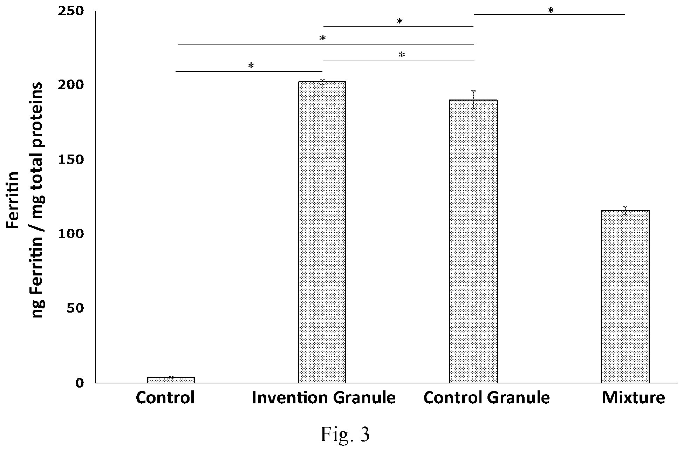

Indeed, contrary to expectations from the intracellular Fe concentration data obtained, while the ferritin induced following exposure to the Mixture was 115 ng ferritin/mg total protein (Figure 3), the cells treated with the Control Granule formulation showed ferritin levels of 189 ng ferritin/mg protein total and, most surprisingly, cells treated with the Invention Granule formulation had ferritin levels of 202 ng ferritin/mg total protein, i.e. approximately 7% higher than the levels observed for the Control Granule.

Reduced expression of ferritin in cells treated with the Mixture formulation indicates that the amount of Fe actually absorbed at cellular level is lower than with the Control Granule formulation, and even more so than with the Invention Granule formulation. As can be seen from this data, the Invention Granule leads to a significant increase with respect to the Control Granule and the Mixture. Such difference is attributed to the presence of copper (Cu), which assists cellular intake of Fe.

Based on the foregoing, the oral administration form of the present invention has proved to be capable of guaranteeing a much higher iron release than the formulation in a simple solid mixture, demonstrating the fact that, upon use of the same composition, the combination of active core and coating layer of the invention is crucial. Furthermore, the presence of copper proved to be even more crucial in increasing intracellular iron absorption compared to the copper-free formulation, as confirmed by the higher ferritin levels present in cells treated with the Invention Granule formulation.

## Claims

1. An oral administration form comprising: a) an active core comprising a solid association of at least one pharmaceutically acceptable Fe(II) salt and at least one pharmaceutically acceptable Cu(II) salt, and (b) an active core-coating layer, said coating layer comprising at least one enteric polymer and at least one hydrophilic polymer, wherein:
- said at least one enteric polymer is cellulose acetate phthalate, cellulose acetate succinate, hydroxypropyl methylcellulose phthalate, hydroxypropyl methylcellulose acetate succinate, polyvinyl acetate phthalate, hydroxyethyl cellulose phthalate, cellulose acetate tetrahydro phthalate, copolymers of methacrylate-methacrylic acid, copolymers of methyl methacrylate- methacrylic acid, sodium alginate, cellulose acetate trimellitate, shellac, or a mixture thereof,
- said at least one hydrophilic polymer is hydroxypropyl methylcellulose, hydroxypropyl cellulose, hydroxyethyl cellulose, xanthan gum, cross-linked carboxy methyl cellulose and the salts thereof, polyvinyl alcohol, polyvinylpyrrolidone, or a mixture thereof,
- the oral administration form further comprises at least one vitamin being at least vitamin C, and
- wherein said at least one pharmaceutically acceptable Fe(II) salt is in the form of micronised particles having a particle size distribution D90 of 1-50 µm, the latter being measured through particle size analysis by sieving with suitable sieves arranged in series, each one of which retains the solid moiety whose granules are larger in size than the sieve holes.

2. The oral administration form of Claim 1, wherein said at least one pharmaceutically acceptable Fe(II) salt is ferrous fumarate, ferrous gluconate, ferrous succinate, ferrous lactate, ferrous citrate, ferrous tartrate, ferrous bisglycinate, ferrous oxalate, or a mixture thereof, and preferably ferrous fumarate.

3. The oral administration form of Claims 1 or 2, wherein said at least one pharmaceutically acceptable Cu(II) salt is copper gluconate, copper citrate, copper succinate, copper salicylate, copper aspartate, or a mixture thereof, and preferably copper gluconate.

4. The oral administration form of any one of Claims 1-3, wherein said at least one pharmaceutically acceptable Fe(II) salt is present in a greater amount than said at least one pharmaceutically acceptable Cu(II) salt, preferably they are in a weight ratio of 200:1 to 2:1, more preferably 100:1 to 10:1, even more preferably from 50:1 to 20:1.

5. The oral administration form of any one of Claims 1-4, wherein the oral administration form comprises up to 10wt% of said at least one pharmaceutically acceptable Fe(II) salt, and preferably up to 5wt%.

6. The oral administration form of any one of Claims 1-5, wherein the oral administration form comprises up to 1wt% of said at least one pharmaceutically acceptable Cu(II) salt, and preferably up to 0. 1wt%.

7. The oral administration form of any one of Claims 1-6, wherein the thickness of the coating layer b) is 100-300 µm, preferably 100-200 µm, more preferably 100-500 µm, and the weight ratio of the active core to the coating layer is of 6:1 to 1:1, preferably 3:1 to 1:1, more preferably 2:1 to 1:1.

8. The oral administration form of any one of Claims 1-7, wherein said at least one pharmaceutically acceptable Fe(II) salt is in the form of micronised particles having a particle size distribution D₉₀ of 2-20 µm.

9. The oral administration form of Claim 8, wherein the active core a) comprises an internal core comprising at least one vitamin coated with a first layer of at least one hydrophilic polymer and a second layer of a solid mixture of at least one pharmaceutically acceptable Fe(II) salt and at least one pharmaceutically acceptable Cu(II) salt.

10. The oral administration form of Claim 8, wherein the active core a) comprises an internal core comprising at least one vitamin, coated with a first layer of at least one hydrophilic polymer, a second layer of at least one pharmaceutically acceptable Cu(II) salt, and a third layer of at least one pharmaceutically acceptable Fe(II) salt.

11. The oral administration form of Claim 8, wherein the active core a) comprises an internal core comprising at least one vitamin, coated with a first layer comprising at least one hydrophilic polymer and at least one pharmaceutically acceptable Fe(II) salt, and a second layer comprising at least one hydrophilic polymer and at least one pharmaceutically acceptable Cu(II) salt.

12. The oral administration form of Claim 8, wherein the active core a) comprises an internal core comprising at least one vitamin, coated with a first layer comprising at least one hydrophilic polymer and at least one pharmaceutically acceptable Fe(II) salt, a second layer of an inert agent, and a third layer comprising at least one hydrophilic polymer and at least one pharmaceutically acceptable Cu(II) salt.

13. The oral administration form of any one of Claims 8-12, in the form of a unit dose comprising up to 200 mg of at least one pharmaceutically acceptable Fe(II) salt, up to 10 mg of at least one pharmaceutically acceptable Cu(II) salt, up to 300 mg of at least one vitamin, and optionally 10-1500 µg of folic acid, said unit dose being preferably in the form of microgranules.

14. The oral administration form of any one of Claims 1-13 for use in the treatment of iron deficiency.

15. A food supplement comprising the oral administration form of any one of Claims 1-13.

## Patentansprüche

1. Orale Darreichungsform, umfassend: a) einen aktiven Kern, umfassend eine feste Assoziation von zumindest einem pharmazeutisch verträglichen Fe(II)-Salz und zumindest einem pharmazeutisch verträglichen Cu(II)-Salz, und (b) eine aktive Kernbeschichtungsschicht, wobei die Beschichtungsschicht zumindest ein enterisches Polymer und zumindest ein hydrophiles Polymer umfasst, wobei:
- das zumindest eine enterische Polymer Celluloseacetatphthalat, Celluloseacetatsuccinat, Hydroxypropylmethylcellulosephthalat, Hydroxypropylmethylcelluloseacetatsuccinat, Polyvinylacetatphthalat, Hydroxyethylcellulosephthalat, Celluloseacetattetrahydrophthalat, Copolymere von Methacrylat-Methacrylsäure, Copolymere von Methylmethacrylat-Methacrylsäure, Natriumalginat, Celluloseacetattrimellitat, Schellack oder eine Mischung davon ist,
- das zumindest eine hydrophile Polymer Hydroxypropylmethylcellulose, Hydroxypropylcellulose, Hydroxyethylcellulose, Xanthangummi, vernetzte Carboxymethylcellulose und die Salze davon, Polyvinylalkohol, Polyvinylpyrrolidon oder eine Mischung davon ist,
- die orale Darreichungsform ferner zumindest ein Vitamin umfasst, das zumindest Vitamin C ist, und
- wobei das zumindest eine pharmazeutisch verträgliche Fe(II)-Salz in der Form von mikronisierten Partikeln mit einer Partikelgrößenverteilung D90 von 1-50 µm ist, wobei letztere durch Partikelgrößenanalyse durch Sieben mit geeigneten Sieben gemessen wird, die in Reihe angeordnet sind, von denen jedes die feste Einheit zurückhält, deren Körner größer als die Sieblöcher sind.

2. Orale Darreichungsform nach Anspruch 1, wobei das zumindest eine pharmazeutisch verträgliche Fe(II)-Salz Eisenfumarat, Eisengluconat, Eisensuccinat, Eisenlactat, Eisencitrat, Eisentartrat, Eisenbisglycinat, Eisenoxalat oder eine Mischung davon und bevorzugt Eisenfumarat ist.

3. Orale Darreichungsform nach Anspruch 1 oder 2, wobei das zumindest eine pharmazeutisch verträgliche Cu(II)-Salz Kupfergluconat, Kupfercitrat, Kupfersuccinat, Kupfersalicylat, Kupferaspartat oder eine Mischung davon und bevorzugt Kupfergluconat ist.

4. Orale Darreichungsform nach einem der Ansprüche 1-3, wobei das zumindest eine pharmazeutisch verträgliche Fe(II)-Salz in einer größeren Menge als das zumindest eine pharmazeutisch verträgliche Cu(II)-Salz vorhanden ist, sie bevorzugt in einem Gewichtsverhältnis von 200:1 bis 2:1, bevorzugter 100:1 bis 10:1, noch bevorzugter von 50:1 bis 20:1 sind.

5. Orale Darreichungsform nach einem der Ansprüche 1-4, wobei die orale Darreichungsform bis zu 10 Gew.-% des zumindest einen pharmazeutisch verträglichen Fe(II)-Salzes und bevorzugt bis zu 5 Gew.-% umfasst.

6. Orale Darreichungsform nach einem der Ansprüche 1-5, wobei die orale Darreichungsform bis zu 1 Gew.-% des zumindest einen pharmazeutisch verträglichen Cu(II)-Salzes und bevorzugt bis zu 0,1 Gew.-% umfasst.

7. Orale Darreichungsform nach einem der Ansprüche 1-6, wobei die Dicke der Beschichtungsschicht b) 100-300 µm, bevorzugt 100-200 µm, bevorzugter 100-500 µm ist und das Gewichtsverhältnis des aktiven Kerns zu der Beschichtungsschicht von 6:1 bis 1:1, bevorzugt 3:1 bis 1:1, bevorzugter 2:1 bis 1:1 ist.

8. Orale Darreichungsform nach einem der Ansprüche 1-7, wobei das zumindest eine pharmazeutisch verträgliche Fe(II)-Salz in der Form von mikronisierten Partikeln mit einer Partikelgrößenverteilung D₉₀ von 2-20 µm ist.

9. Orale Darreichungsform nach Anspruch 8, wobei der aktive Kern a) einen Innenkern umfassend zumindest ein Vitamin, beschichtet mit einer ersten Schicht von zumindest einem hydrophilen Polymer und einer zweiten Schicht einer festen Mischung von zumindest einem pharmazeutisch verträglichen Fe(II)-Salz und zumindest einem pharmazeutisch verträglichen Cu(II)-Salz umfasst.

10. Orale Darreichungsform nach Anspruch 8, wobei der aktive Kern a) einen Innenkern umfassend zumindest ein Vitamin, beschichtet mit einer ersten Schicht von zumindest einem hydrophilen Polymer, einer zweiten Schicht von zumindest einem pharmazeutisch verträglichen Cu(II)-Salz und einer dritten Schicht von zumindest einem pharmazeutisch verträglichen Fe(II)-Salz umfasst.

11. Orale Darreichungsform nach Anspruch 8, wobei der aktive Kern a) einen Innenkern umfassend zumindest ein Vitamin, beschichtet mit einer ersten Schicht umfassend zumindest ein hydrophiles Polymer und zumindest ein pharmazeutisch verträgliches Fe(II)-Salz und einer zweiten Schicht umfassend zumindest ein hydrophiles Polymer und zumindest ein pharmazeutisch verträgliches Cu(II)-Salz umfasst.

12. Orale Darreichungsform nach Anspruch 8, wobei der aktive Kern a) einen Innenkern umfassend zumindest ein Vitamin, beschichtet mit einer ersten Schicht umfassend zumindest ein hydrophiles Polymer und zumindest ein pharmazeutisch verträgliches Fe(II)-Salz, einer zweiten Schicht eines inerten Mittels und einer dritten Schicht umfassend zumindest ein hydrophiles Polymer und zumindest ein pharmazeutisch verträgliches Cu(II)-Salz umfasst.

13. Orale Darreichungsform nach einem der Ansprüche 8-12 in der Form einer Einheitsdosis umfassend bis zu 200 mg von zumindest einem pharmazeutisch verträglichen Fe(II)-Salz, bis zu 10 mg von zumindest einem pharmazeutisch verträglichen Cu(II)-Salz, bis zu 300 mg von zumindest einem Vitamin und optional 10-1500 µg Folsäure, wobei die Einheitsdosis bevorzugt in der Form von Mikrogranulat ist.

14. Orale Darreichungsform nach einem der Ansprüche 1-13 zur Verwendung bei der Behandlung von Eisenmangel.

15. Nahrungsergänzungsmittel, umfassend die orale Darreichungsform nach einem der Ansprüche 1-13.

## Revendications

1. Forme à administrer par voie orale comprenant : a) un noyau actif comprenant une association solide d'au moins un sel de Fe(II) pharmaceutiquement acceptable et d'au moins un sel de Cu(II) pharmaceutiquement acceptable, et (b) une couche d'enrobage de noyau actif, ladite couche d'enrobage comprenant au moins un polymère entérique et au moins un polymère hydrophile :
- ledit au moins un polymère entérique étant du phtalate d'acétate de cellulose, du succinate d'acétate de cellulose, du phtalate d'hydroxypropylméthylcellulose, du succinate d'acétate d'hydroxypropylméthylcellulose, du phtalate d'acétate de polyvinyle, du phtalate d'hydroxyéthylcellulose, du phtalate de tétrahydroacétate de cellulose, des copolymères de méthacrylate-acide méthacrylique, des copolymères de méthacrylate de méthyle-acide méthacrylique, de l'alginate de sodium, du trimellitate d'acétate de cellulose, de la gomme laque, ou un mélange de ceux-ci,
- ledit au moins un polymère hydrophile étant de l'hydroxypropylméthylcellulose, l'hydroxypropylcellulose, de l'hydroxyéthylcellulose, de la gomme xanthane, de la carboxyméthylcellulose réticulée et ses sels, de l'alcool polyvinylique, de la polyvinylpyrrolidone ou un mélange de ceux-ci,
- ladite forme à administrer par voie orale comprenant en outre au moins une vitamine qui est au moins la vitamine C, et
- ledit au moins un sel de Fe(II) pharmaceutiquement acceptable se présentant sous la forme de particules micronisées ayant une distribution granulométrique D90 de 1 à 50 µm, cette dernière étant mesurée par analyse granulométrique par tamisage avec des tamis appropriés disposés en série, chacun desquels conservant la fraction solide dont les granules sont plus gros que les trous de tamis.

2. Forme à administrer par voie orale selon la revendication 1, ledit au moins un sel de Fe(II) pharmaceutiquement acceptable étant du fumarate ferreux, du gluconate ferreux, du succinate ferreux, du lactate ferreux, du citrate ferreux, du tartrate ferreux, du bisglycinate ferreux, de l'oxalate ferreux ou un mélange de ceux-ci, et de préférence du fumarate ferreux.

3. Forme à administrer par voie orale selon la revendication 1 ou 2, ledit au moins un sel de Cu(II) pharmaceutiquement acceptable étant du gluconate de cuivre, du citrate de cuivre, du succinate de cuivre, du salicylate de cuivre, de l'aspartate de cuivre, ou un mélange de ceux-ci, et de préférence du gluconate de cuivre.

4. Forme à administrer par voie orale selon l'une quelconque des revendications 1 à 3, ledit au moins un sel de Fe(II) pharmaceutiquement acceptable étant présent en une quantité supérieure audit au moins un sel de Cu(II) pharmaceutiquement acceptable, de préférence se trouvant dans un rapport pondéral de 200:1 à 2:1, plus préférablement de 100:1 à 10:1, encore plus préférablement de 50:1 à 20:1.

5. Forme à administrer par voie orale selon l'une quelconque des revendications 1 à 4, ladite forme à administrer par voie orale comprenant jusqu'à 10 % en poids dudit au moins un sel de Fe(II) pharmaceutiquement acceptable, et de préférence jusqu'à 5 % en poids.

6. Forme à administrer par voie orale selon l'une quelconque des revendications 1 à 5, ladite forme à administrer par voie orale comprenant jusqu'à 1 % en poids dudit au moins un sel de Cu(II) pharmaceutiquement acceptable, et de préférence jusqu'à 0,1 % en poids.

7. Forme à administrer par voie orale selon l'une quelconque des revendications 1 à 6, ladite épaisseur de la couche d'enrobage b) étant de 100 à 300 µm, de préférence de 100 à 200 µm, plus préférablement de 100 à 500 µm, et ledit rapport pondéral du noyau actif à la couche d'enrobage étant de 6:1 à 1:1, de préférence de 3:1 à 1:1, plus préférablement de 2:1 à 1:1.

8. Forme à administrer par voie orale selon l'une quelconque des revendications 1 à 7, ledit au moins un sel de Fe(II) pharmaceutiquement acceptable se présentant sous la forme de particules micronisées ayant une distribution granulométrique D₉₀ de 2 à 20 µm.

9. Forme à administrer par voie orale selon la revendication 8, ledit noyau actif a) comprenant un noyau interne comprenant au moins une vitamine enrobé d'une première couche d'au moins un polymère hydrophile et d'une seconde couche d'un mélange solide d'au moins un sel de Fe(II) pharmaceutiquement acceptable et d'au moins un sel de Cu(II) pharmaceutiquement acceptable.

10. Forme à administrer par voie orale selon la revendication 8, ledit noyau actif a) comprenant un noyau interne comprenant au moins une vitamine, enrobé d'une première couche d'au moins un polymère hydrophile, d'une deuxième couche d'au moins un sel de Cu(II) pharmaceutiquement acceptable et d'une troisième couche d'au moins un sel de Fe(II) pharmaceutiquement acceptable.

11. Forme à administrer par voie orale selon la revendication 8, ledit noyau actif a) comprenant un noyau interne comprenant au moins une vitamine, enrobé d'une première couche comprenant au moins un polymère hydrophile et au moins un sel de Fe(II) pharmaceutiquement acceptable, et d'une seconde couche comprenant au moins un polymère hydrophile et au moins un sel de Cu(II) pharmaceutiquement acceptable.

12. Forme à administrer par voie orale selon la revendication 8, ledit noyau actif a) comprenant un noyau interne comprenant au moins une vitamine, enrobé d'une première couche comprenant au moins un polymère hydrophile et au moins un sel de Fe(II) pharmaceutiquement acceptable, d'une deuxième couche d'un agent inerte et d'une troisième couche comprenant au moins un polymère hydrophile et au moins un sel de Cu(II) pharmaceutiquement acceptable.

13. Forme à administrer par voie orale selon l'une quelconque des revendications 8 à 12, sous la forme d'une dose unitaire comprenant jusqu'à 200 mg d'au moins un sel de Fe(II) pharmaceutiquement acceptable, jusqu'à 10 mg d'au moins un sel de Cu(II) pharmaceutiquement acceptable, jusqu'à 300 mg d'au moins une vitamine et éventuellement 10 à 1500 µg d'acide folique, ladite dose unitaire se présentant de préférence sous la forme de microgranules.

14. Forme à administrer par voie orale selon l'une quelconque des revendications 1 à 13, destinée à être utilisée dans le traitement d'une carence en fer.

15. Complément alimentaire comprenant la forme à administrer par voie orale selon l'une quelconque des revendications 1 à 13.
